# FASCICULE DE BREVET EUROPEEN

(11) **EP 3 113 673 B1**
(45) Date de publication et mention de la délivrance du brevet: **01.04.2020**
(21) Numéro de dépôt: 15709254.5
(22) Date de dépôt: 16.02.2015
(51) Int. Cl.: A61B 5/00, A61G 10/00, G01N 15/02, A61M 11/00

(54) **SYSTEME D'EXPOSITION AUX ALLERGENES COMPRENANT UNE CHAMBRE DE MELANGE ENTRE L'AIR ET LES ALLERGENES, SEPAREE DE LA SALLE D'EXPOSITION ACCUEILLANT LES PATIENTS**
ALLERGENEXPOSITIONSSYSTEM MIT EINER KAMMER ZUR MISCHUNG VON LUFT UND ALLERGENEN, DIE VOM EXPOSITIONSRAUM GETRENNT IST, IN DEM DIE PATIENTEN UNTERGEBRACHT SIND
ALLERGEN EXPOSURE SYSTEM COMPRISING A CHAMBER FOR MIXING AIR AND ALLERGENS, WHICH IS SEPARATED FROM THE EXPOSURE ROOM THAT ACCOMMODATES THE PATIENTS

(30) Priorité: 03.03.2014 FR 1451669
(43) Date de publication de la demande: 11.01.2017
(73) Titulaire: Alyatec, 67000 Strasbourg (FR)
(72) Inventeur: SANTAILLER, Gérard, F-69480 Marcy (FR)
(74) Mandataire: Merckling, Norbert
(86) Numéro de dépôt international: PCT/FR2015/050366
(87) Numéro de publication internationale: WO 2015/132497

(56) Documents cités:
- W. EDUARD ET AL: "Generation and Homogeneity of Aerosols in a Human Whole-Body Inhalation Chamber", ANNALS OF OCCUPATIONAL HYGIENE, vol. 52, no. 6, 7 juillet 2008 (2008-07-07), pages 545-554, XP055134071, ISSN: 0003-4878, DOI: 10.1093/annhyg/men039
- C. LIDÈN ET AL: "A New Whole-body Exposure Chamber for Human Skin and Lung Challenge Experiments-the Generation of Wheat Flour Aerosols", ANNALS OF OCCUPATIONAL HYGIENE, vol. 42, no. 8, 1 novembre 1998 (1998-11-01), pages 541-547, XP055134038, ISSN: 0003-4878, DOI: 10.1093/annhyg/42.8.541
- CHRISTIAN MONSÉ ET AL: "Considerations for the design and technical setup of a human whole-body exposure chamber", INHALATION TOXICOLOGY, vol. 24, no. 2, 1 janvier 2012 (2012-01-01), pages 99-108, XP055134058, ISSN: 0895-8378, DOI: 10.3109/08958378.2011.640362
- J. H. DAY ET AL: "The role of allergen challenge chambers in the evaluation of anti-allergic medication: an international consensus paper", CLINICAL <HTML_ENT GLYPH="@AMP;" ASCII="&"/> EXPERIMENTAL ALLERGY REVIEWS, vol. 6, no. 2, 1 février 2006 (2006-02-01) , pages 31-59, XP055133981, ISSN: 1472-9725, DOI: 10.1111/j.1365-2222.2005.00099.x
- P. P. H LE BRUN ET AL: "A review of the technical aspects of drug nebulization", PHARMACY WORLD AND SCIENCE, vol. 22, no. 3, 1 janvier 2000 (2000-01-01), pages 75-81, XP055134023,

## Description

La présente invention concerne un système d'exposition aux allergènes comprenant une salle d'exposition destinée à accueillir des patients et dans laquelle on réalise une atmosphère d'inhalation à teneur contrôlée en allergènes en vue d'une provocation allergique chez ces patients.

La salle d'exposition est une enceinte confinée, de type dit EEC (appelée Environmental Exposure Chamber ou European Exposure Chamber), connue également sous le nom de chambre de test d'allergie.

Les allergies sont un fléau mondial qui concerne plus d'une personne sur quatre en Occident. On estime que d'ici quelques années, près de 50 % de la population des pays développés pourrait être touchée par au moins une maladie allergique. La recherche médicale dans le domaine de l'allergie et notamment celle se rapportant au développement de médicaments antiallergiques ou de traitements de désensibilisation, est de ce fait un secteur qui se développe considérablement.

Pour réaliser des études cliniques relatives à l'allergie ou pour évaluer l'efficacité de nouveaux médicaments ou de traitement de désensibilisation, il est indispensable d'observer la réaction de patients allergiques lorsqu'ils se retrouvent exposés aux allergènes naturels.

La réalisation de telles observations en milieu naturel, de façon scientifique et objective, est très difficile en raison des importantes fluctuations de la quantité d'allergènes inhalés par un patient en fonction, par exemple, de la saison, des conditions climatiques ou des endroits fréquentés par le patient. En effet, la quantité d'allergènes naturellement présents dans l'air ambiant est très variable selon par exemple la région où l'on se trouve, la saison, les conditions météorologiques notamment la température, l'humidité ou la présence de vent, le moment de la journée, ou même la hauteur par rapport au niveau du sol.

Pour pouvoir s'affranchir de ces très nombreux paramètres qui fluctuent de façon difficilement contrôlable, on a développé dans l'art antérieur des dispositifs appelés chambres de test d'allergie ou EEC (European/Environmental Exposure Chamber).

Ces dispositifs permettent d'accueillir un ou plusieurs patients dans un lieu clos où l'on diffuse une quantité contrôlée d'allergènes et d'observer scientifiquement leur réaction physiologique après un temps d'exposition plus ou moins long. Les différents paramètres expérimentaux au sein de la chambre sont constants et contrôlés tout au long de l'expérimentation. On peut ainsi réaliser des tests dans des conditions reproductibles sur plusieurs patients ou plusieurs fois sur un même patient et obtenir immédiatement et de façon fiable des résultats comparables les uns avec les autres.

De tels dispositifs rendent possibles des tests d'inhalation d'allergènes sous conditions contrôlées proches de l'état naturel et reproductibles. Ils garantissent des résultats fiables, complets, comparables et utilisables scientifiquement dans le cadre de nombreuses études cliniques. Ils sont ainsi utilisables par exemple pour tester in vivo l'efficacité de traitements de désensibilisation ou de nouveaux médicaments antiallergiques et par exemple pour déterminer les doses à prescrire ou la durée d'efficacité.

On connaît ainsi par exemple la chambre de test d'allergie décrite dans le brevet européen n° EP 1335750 au nom de HORAK ou celles décrites dans les demandes de brevet FRAUNHOFER WO 2010/0063714 et PATEL WO 2007/140601.

Dans ces dispositifs antérieurs connus, une préoccupation constante concerne l'obtention d'une concentration en allergènes homogène dans toute la salle d'exposition. En effet, pour obtenir des résultats fiables et exploitables, il est très important de s'assurer que les patients sont exposés à la même concentration d'allergènes quelle que soit leur position dans la salle. Différents dispositifs de distribution et de répartition des allergènes dans la salle d'exposition ont donc été imaginés à cet effet dans l'art antérieur.

Dans le brevet HORAK, on introduit d'une part une poudre d'allergène au moyen d'un distributeur de particules à air comprimé placé dans le plafond de la salle d'exposition et d'autre part, de l'air frais dépourvu d'allergènes au moyen de buses sphériques, distinctes du distributeur de particules, également situées au plafond mais à des endroits différents et orientées dans des directions différentes afin de provoquer un courant d'air turbulent.

Dans les brevets FRAUNHOFER et PATEL, on utilise une préparation liquide d'allergènes à partir de laquelle on génère un aérosol. Une fois introduit dans la salle d'exposition, cet aérosol est mélangé, au moyen d'un ou plusieurs ventilateurs, à l'air ambiant se trouvant dans la salle d'exposition et provenant d'entrées d'air distinctes.

Dans tous ces dispositifs, le mélange entre l'air et les particules d'allergènes se fait directement dans la salle d'exposition au moyen d'un fort courant d'air turbulent ou de ventilateurs.

De tels systèmes de distributions ne sont pas satisfaisants et ne permettent pas de garantir un mélange uniforme et une concentration homogène dans l'ensemble de la salle d'exposition lorsque celle-ci est de grande taille. La concentration est différente en fonction de la hauteur par rapport au niveau du sol, de l'éloignement par rapport à la zone d'entrée des allergènes ou de la position par rapport aux ventilateurs.

De plus, les turbulences importantes et les forts courants d'air générés par les buses et/ou les ventilateurs sont désagréables pour les patients se trouvant dans la salle d'exposition et risquent en outre de provoquer une dégradation des allergènes, qui sont des composés particulièrement fragiles, ou un réarrangement des particules par agrégation modifiant leur taille.

On connait également les publications suivantes : W. EDUARD ET AL. « Génération and Homogeneity of Aérosol in a Human Whole-Body Inhalation Chamber », C. LINDEN ET AL. "A New Whole-body Exposure Chamber for Human Skin and Lung Challenge Experiments - the Génération of Wheat Flour Aérosol" et CHRISTIAN MONSE ET AL. "Considérations for the design and technical setup of a human whole-body exposure chamber".

Ces documents décrivent des systèmes d'exposition respectivement à de l'oxyde d'aluminium, de la farine de blé et à des gaz. Dans ces systèmes, le mélange avec l'air de ventilation ne se fait plus dans la salle d'exposition, mais en amont de celle-ci, directement dans le conduit d'arrivée d'air, que ce soit au moyen d'un conduit intérieur coaxial débouchant longitudinalement dans le conduit d'air comme dans le premier document, ou au moyen d'une simple connexion en T comme dans les deux autres documents.

Le mélange obtenu avec ces dispositifs antérieurs n'est pas non plus satisfaisant car le flux d'air circule trop rapidement dans le conduit d'arrivée pour qu'un véritable mélange homogène se produise. Les particules sont simplement aspirées par effet Venturi et entraînées sans mélange réel entre les différents flux.

Le dispositif selon l'invention permet au contraire de garantir un bien meilleur mélange entre l'air et les particules d'allergènes, conduisant à une homogénéisation nettement supérieure de la concentration d'allergènes dans l'ensemble de la salle d'exposition et ce avec beaucoup moins de risque d'endommager les particules d'allergènes.

Pour cela, l'invention enseigne un système d'exposition aux allergènes comprenant un dispositif d'injection d'allergènes, un conduit d'arrivée d'air et une salle d'exposition qui contient de l'air chargé en particules d'allergènes obtenu par mélange d'un flux de particules d'allergènes provenant du dispositif d'injection d'allergènes et d'un flux d'air dépourvu d'allergènes amené par le conduit d'arrivée d'air, cette salle d'exposition étant destinée à accueillir des patients venant inhaler l'air chargé en particules d'allergènes en vue d'une provocation allergique.

Selon l'invention, le système d'exposition aux allergènes comprend également une chambre de mélange, délimitée par des parois, distincte du conduit d'arrivée d'air et de la salle d'exposition, dans laquelle se produit ledit mélange entre le flux de particules d'allergènes et le flux d'air dépourvu d'allergènes.

Cette chambre de mélange, située en amont de la salle d'exposition, est un volume creux délimité par des parois de forme et de composition adaptées pour ne pas risquer de détériorer les particules d'allergènes.

Elle comporte au moins une première entrée, appelée entrée d'allergènes, qui communique avec la sortie du dispositif d'injection d'allergènes et par laquelle pénètre le flux de particules d'allergènes sous la forme d'un nébulisat. Cette première entrée est adaptée pour ne pas dégrader les allergènes qui la traversent. Elle ne comporte, par exemple, pas de buses ou de système agressif susceptible d'endommager les allergènes. Il s'agit de préférence d'un ensemble d'une ou plusieurs ouvertures simples. De préférence, cette entrée communique directement avec la sortie du dispositif d'injection d'allergènes ou au moyen d'un conduit droit de faible longueur. La chambre de mélange comporte également au moins une deuxième entrée, appelée entrée d'air, qui communique avec le conduit d'arrivée d'air et par laquelle pénètre le flux d'air dépourvu d'allergènes qui est destiné à venir ventiler la salle d'exposition. Cette deuxième entrée est préférentiellement équipée d'un dispositif de filtration, de préférence un système de filtration à haute efficacité, afin de garantir que l'air qui pénètre dans la chambre de mélange est dépourvu d'allergènes autres que ceux volontairement introduits par la première entrée.

La chambre de mélange comporte enfin au moins une bouche de diffusion qui communique avec la salle d'exposition et par laquelle s'échappe en direction de la salle d'exposition un flux d'air chargé en particules d'allergènes, obtenu par le mélange dans la chambre de mélange du flux d'air dépourvu d'allergènes et du flux de particules d'allergènes. il s'agit de préférence d'une ouverture ménagée dans la paroi de la chambre de mélange qui débouche dans la salle d'exposition ou dans un conduit ouvert allant de la chambre de mélange à la salle d'exposition.

A proximité de l'entrée d'air, la chambre de mélange selon l'invention, forme un élargissement par rapport au conduit d'arrivée d'air qui provoque une expansion du flux d'air dépourvu d'allergènes lorsqu'il pénètre dans la chambre.

De ce fait, la vitesse de l'air est ralentie lorsqu'il pénètre dans la chambre de mélange et il ne se crée que des miro-turbulences d'intensité réduite. On obtient ainsi un mélange plus efficace du flux d'air avec le flux des particules d'allergènes qui ont le temps de s'interpénétrer et de se mélanger intimement avant d'arriver au niveau de la bouche de diffusion.

Ce mélange est également beaucoup plus doux, ce qui limite le risque d'endommagement des particules fragiles d'allergènes et ne provoque que très peu de réarrangements des particules par agrégation, qui étaient fréquemment rencontrés avec les dispositifs antérieurs à brassages ou turbulences importantes. La taille des particules inhalées par les patients présents dans la salle d'exposition est beaucoup plus stable et homogène et peut ainsi être mieux contrôlée.

A proximité de la bouche de diffusion, la section de la chambre de mélange peut préférentiellement diminuer de manière à former un rétrécissement.

Le flux d'air chargé en particules d'allergènes est ainsi accéléré par compression juste avant sa sortie de la chambre de mélange. Il pénètre ainsi dans la salle d'exposition avec une vitesse suffisante pour garantir le portage des particules d'allergènes dans la totalité de la salle d'exposition.

Dans la chambre de mélange, la première entrée et la deuxième entrée peuvent avantageusement être disposées l'une par rapport à l'autre de manière que l'air qui pénètre par la deuxième entrée vienne balayer la première entrée et ainsi entraîner les particules d'allergènes arrivant par la première entrée et qu'il se crée des micro turbulences au sein de la chambre de mélange permettant d'obtenir un excellent mélange entre les deux flux et ainsi de garantir la sortie à travers l'au moins une bouche de diffusion d'un flux d'air chargé en particules d'allergènes homogène et de concentration sensiblement constante.

Selon une variante préférentielle de l'invention, la première entrée et la deuxième entrée de la chambre de mélange sont disposées de façon sensiblement perpendiculaire l'une par rapport à l'autre.

Le dispositif d'injection d'allergènes est un nébuliseur, un atomiseur par onde capillaire, et le flux de particules d'allergènes est un nébulisat.

Selon un mode de réalisation de l'invention, la chambre de mélange peut comporter en outre un rétrécissement progressif suivi d'un agrandissement progressif de sa largeur, créant ainsi une zone d'étranglement dans laquelle se trouve l'entrée d'allergènes.

Selon un mode de réalisation de l'invention, la chambre de mélange peut être située au moins partiellement au-dessus de la salle d'exposition.

Grâce à la chambre de mélange selon l'invention, on obtient un excellent mélange entre les deux flux et on maitrise la concentration en allergènes du flux résultant qui sort de la chambre de mélange et entre dans la salle d'exposition. Le mélange est remarquable, sans risque de dégradation des allergènes et les turbulences, limitées à des micro-turbulences, sont localisées à l'intérieur de la chambre de mélange ce qui améliore considérablement le confort des patients présents dans la salle d'exposition.

Selon la taille et la forme de la salle d'exposition, la chambre de mélange comporte un nombre adapté de bouches de diffusion disposées de façon à garantir une diffusion homogène de l'air chargé en allergènes dans la salle d'exposition, sans direction préférentielle.

D'autres caractéristiques et avantages de l'invention apparaitront à la lecture de la description détaillée qui va suivre, description faite en référence aux dessins annexés, dans lesquels :
- la figure 1 est une vue de dessus schématique d'ensemble d'un exemple de système d'exposition aux allergènes selon l'invention ;
- la figure 2 est une vue schématique en coupe transversale d'un exemple de système d'exposition aux allergènes selon l'invention ;
- la figure 3 est une vue schématique en coupe d'un exemple de chambre de mélange pour un système d'exposition aux allergènes selon l'invention ;
- la figure 4 est une vue en coupe transversale d'un exemple de chambre de mélange et de salle d'exposition pour un système d'exposition aux allergènes selon l'invention ;
- la figure 5 est une vue de dessus d'un exemple de chambre de mélange et de salle d'exposition correspondant à celui de la figure 4, pour un système d'exposition aux allergènes selon l'invention, et
- les figures 6 et 7 sont des vues de dessus de deux autres exemples de chambre de mélange selon l'invention.

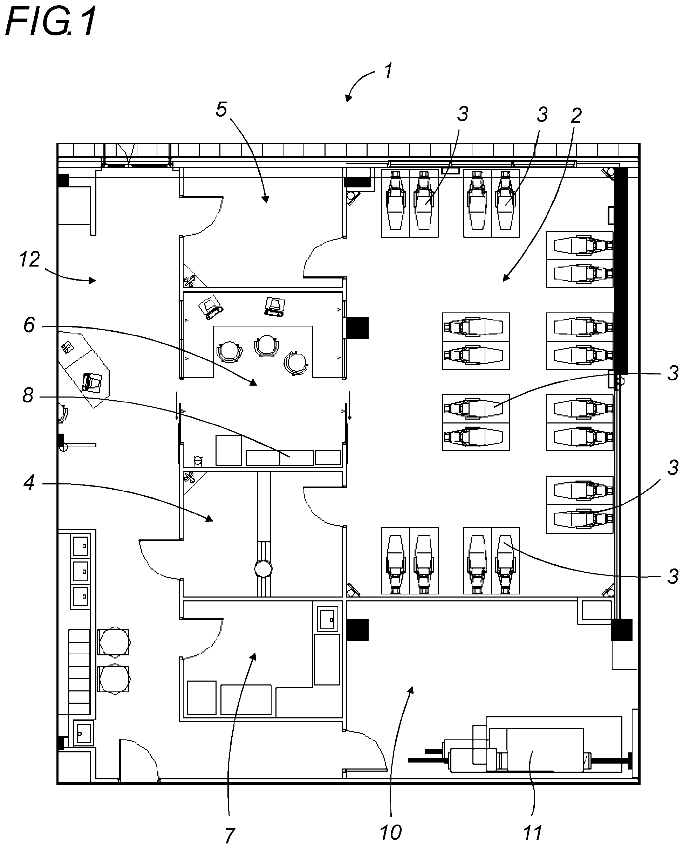

Le système d'exposition aux allergènes selon la présente invention va maintenant être décrit de façon détaillée en référence aux figures 1 à 7. Les éléments équivalents représentés sur les différentes figures porteront les mêmes références numériques.

On a représenté sur la figure 1 le plan général d'un exemple de système d'exposition aux allergènes 1 selon l'invention qui comprend plusieurs pièces.

Le système d'exposition aux allergènes 1 comprend une salle d'exposition 2 destinée à accueillir les patients à observer et dans laquelle on peut produire une atmosphère d'inhalation à teneur contrôlée en allergènes.

La salle d'exposition 2 est une enceinte confinée, dont la pression ambiante est inférieure à la pression de référence (qui est celle des locaux environnants le système d'exposition 1) et à celle du reste du système d'exposition 1.

La taille et la forme de la salle d'exposition 2 dépendent du lieu d'implantation du système, des contraintes techniques à respecter et des souhaits des exploitants.

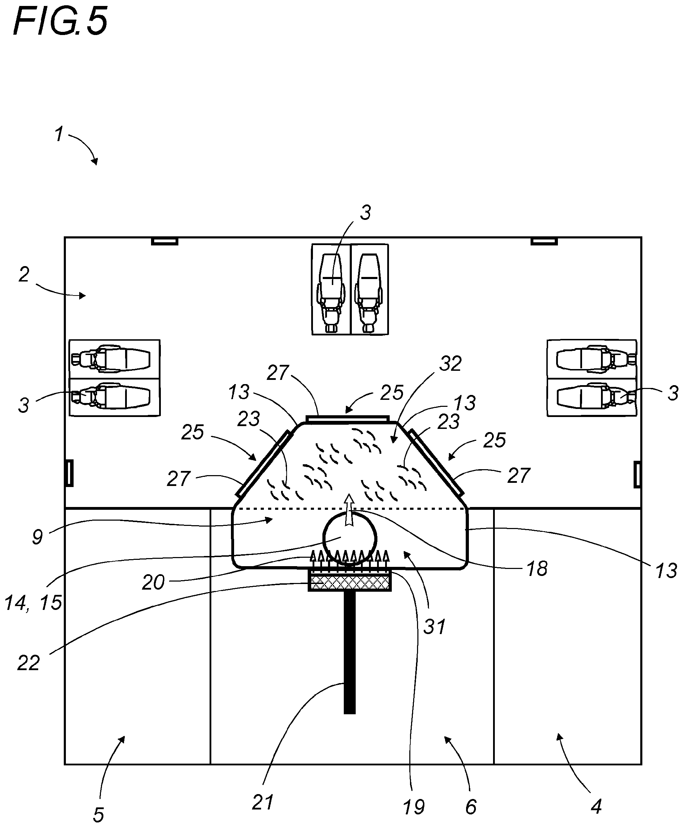

Elle renferme un ou plusieurs sièges 3 dans lesquels les patients peuvent s'installer confortablement pour toute la durée de l'exposition. A titre d'exemple non limitatif, la salle d'exposition 2 représentée sur la figure 1 comporte vingt sièges 3 répartis par groupe de deux dans toute la salle, celle représentée sur la figure 5 est plus petite et ne comporte que six sièges 3.

Un sas d'entrée 4 et un sas de sortie 5, en surpression par rapport à la salle d'exposition 2 et par rapport à l'extérieur, permettent aux patients d'entrer et de sortir de la salle d'exposition 2 sans qu'il y ait de contamination par les allergènes à l'extérieur du système d'exposition aux allergènes 1. Ces sas 4 et 5 permettent également d'empêcher les polluants extérieurs (autres allergènes, composés chimiques) d'entrer dans la salle d'exposition 2.

Une salle de contrôle 6, également en surpression par rapport à la salle d'exposition 2 et par rapport à l'extérieur, permet aux opérateurs de venir régler et contrôler les différents paramètres d'expérimentation et de surveiller les patients présents dans la salle d'exposition 2.

La composition d'allergènes destinée à être inhalée par les patients est par exemple préparée dans un laboratoire 7 équipé de manière à permettre la préparation en toute sécurité pour les techniciens et les patients. Elle est ensuite placée dans un dispositif d'injection d'allergènes 8 qui l'envoie dans une chambre de mélange 9 non représentée sur la figure 1 mais qui sera largement décrite par la suite en référence aux figures suivantes.

Le système d'exposition aux allergènes 1 peut comprendre en outre un local technique 10 dans lequel se trouvent par exemple les appareils 11 nécessaires à la ventilation, l'humidification, la climatisation et/ou le chauffage des différentes pièces du système d'exposition aux allergènes 1 ou tout autre appareillage ou matériel encombrant nécessaire au fonctionnement du système 1.

L'ensemble pourra être complété par une salle d'accueil et d'attente 12 pour les patients.

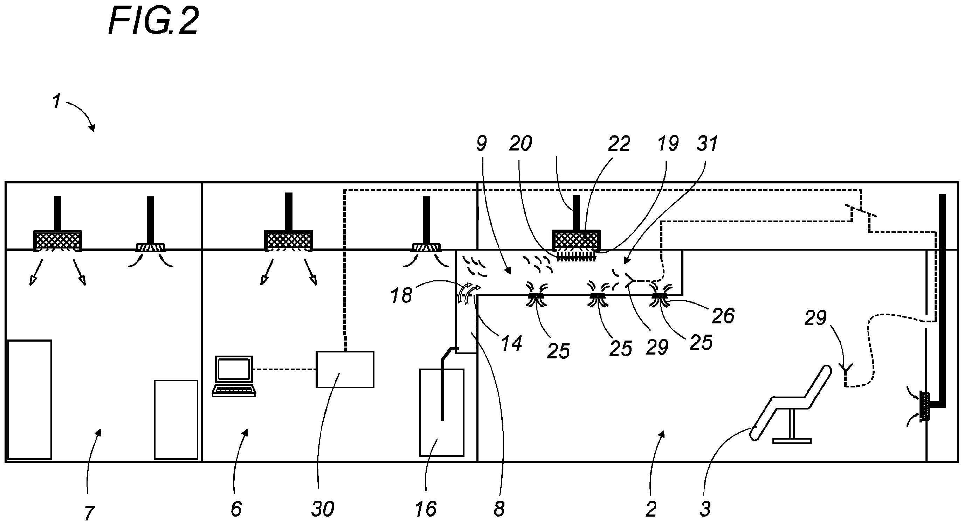

Selon l'invention, le système d'exposition aux allergènes 1 comprend une chambre de mélange 9 dont plusieurs exemples ont été représentés sur les figures 2 à 7.

Il s'agit d'un volume creux, délimité par des parois 13 qui forment un caisson, distinct et séparé de la salle d'exposition 2 et également distinct et séparé du conduit d'arrivée d'air, dans lequel on réalise le mélange entre les particules d'allergènes et un flux d'air dépourvu d'allergènes, avant leur introduction dans la salle d'exposition 2.

Pour ne pas endommager les particules d'allergènes et pour faciliter le nettoyage entre deux sessions d'exposition, les parois 13 de la chambre de mélange 9 sont préférentiellement lisses, sans parties saillantes agressive, ni angles vifs ou recoins propices aux accumulations de matières.

Elles sont de préférence réalisées dans un matériau qui ne relargue pas ou peu les particules et les composés organiques volatils (COV), et qui est facilement nettoyable et résistant aux produits utilisés pour le nettoyage (H₂O₂ par exemple). On peut par exemple utiliser pour cela des panneaux stratifiés haute pression HPL (laminés haute pression) thermodurcissables, tels que les massifs compacts par exemple et notamment ceux commercialisés sous le nom de FUNDERMAX® ou ATHLON®. Il s'agit d'un matériau formé de panneaux composites à base de papier imprégné de résine phénolique et de résine mélamine, ignifugés dans la masse. On peut également utiliser par exemple une résine acrylique à charges minérales, notamment celle commercialisée sous le nom de CORIAN®, HI-MAX® ou STARON®.

Dans cette demande de brevet, on définira un « matériau qui relargue peu de particules » comme étant un matériau relarguant un nombre de particules inférieur à ce qui est requis pour obtenir un classement ISO inférieur ou égal à 7 selon la norme 14644-1.

La chambre de mélange 9 comporte au moins une entrée, appelée entrée d'allergènes 14, par laquelle pénètrent les particules d'allergènes destinées à être inhalées par les patients.

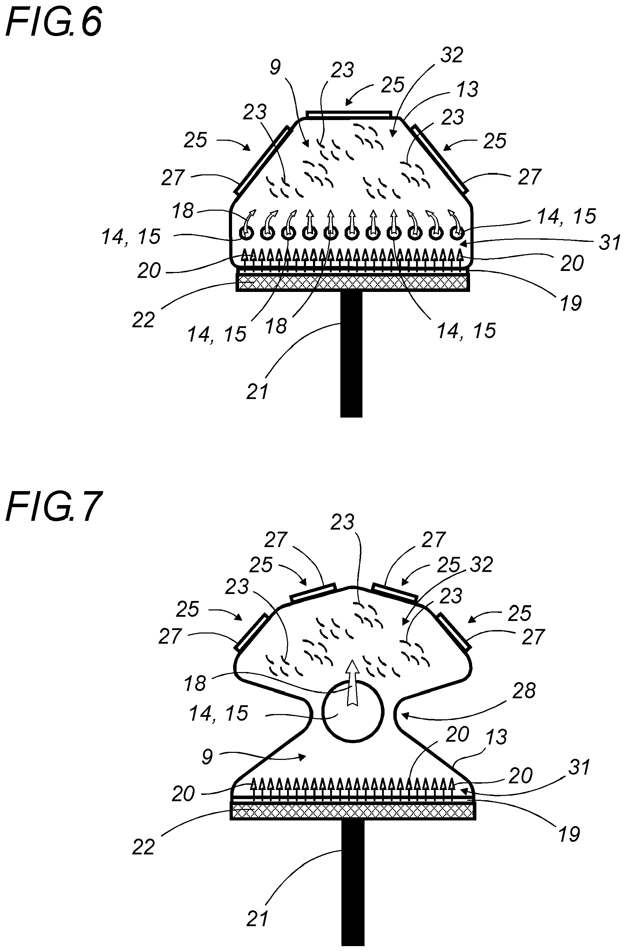

Cette entrée d'allergènes 14 est adaptée pour ne pas dégrader les allergènes qui la traversent. Elle ne comporte pas de buses ou de système agressif susceptible d'endommager les allergènes. Il peut s'agir notamment d'une simple ouverture 15, par exemple circulaire comme représenté sur les figures 5 et 7.

L'entrée d'allergènes 14 communique avec la sortie du dispositif d'injection d'allergènes 8, de préférence directement comme représenté, ou par exemple au moyen d'un conduit droit de faible longueur.

D'autres formes de liaison moins favorables pour préserver l'intégrité des particules d'allergènes sont évidemment possibles, notamment un conduit d'amenée coudé ou de plus grande longueur.

Selon une autre variante de l'invention, la chambre de mélange peut comporter plusieurs entrées d'allergènes 14, par exemple sous la forme d'un ensemble d'ouvertures 15 sensiblement alignées à la manière d'une rampe d'injection comme représenté sur l'exemple de la figure 6. D'autres dispositions de ces entrées d'allergènes 14 sont évidemment possibles sans sortir de la portée de la présente invention.

Le dispositif d'injection d'allergènes 8 est un atomiseur par ondes capillaires.

Sur les exemples représentés, la composition allergénique liquide préparée dans le laboratoire 7 est versée dans un réservoir 16, puis envoyée dans le dispositif d'injection d'allergène 8 par la conduite 17. Elle est ensuite injectée dans la chambre de mélange 9 dans laquelle elle pénètre à travers la ou les ouverture(s) 15 qui constitue(nt) l'entrée d'allergènes 14 (phénomène d'admission).

Sur les figures, le flux de particules d'allergènes 18 a été symbolisé par des flèches blanches. Il s'agit d'un nébulisat.

La chambre de mélange comporte également au moins une entrée d'air 19 par laquelle arrive de l'air dépourvu d'allergènes destiné à venir ventiler la salle d'exposition 2. Ce flux d'air 20, laminaire et dépourvu d'allergènes a été symbolisé sur les figures par des flèches noires à pointe blanche.

Cette entrée d'air 19 communique avec un conduit d'arrivée d'air 21.

Un dispositif de filtration 22 est préférentiellement disposé au niveau de l'entrée d'air 19, ou interposé entre cette entrée d'air 19 et le conduit d'arrivée d'air 21, de manière à empêcher que d'éventuels allergènes ou autres particules indésirables, et éventuellement même certains polluants chimiques, présents dans l'air arrivant par le conduit d'arrivé d'air 21 ne pénètrent dans la chambre de mélange 9 et par la suite dans la salle d'exposition 2.

Le dispositif de filtration 22 fonctionne dans les deux sens, ce qui garantit en outre que les particules d'allergènes présentes dans la chambre de mélange 9 ne peuvent pas ressortir par le conduit d'arrivée d'air 21 en cas de coupure du flux d'air. Une rétro-contamination est ainsi évitée et le confinement de la salle d'exposition 2 est assuré.

Le dispositif de filtration 22 comprend préférentiellement un dispositif de filtration à haute efficacité, également appelé filtre HEPA (High Efficiency Particulate Air filter), ou un dispositif de filtration à très haute efficacité, également appelé filtre ULPA (Ultra Low Particulate/Penetration Air filter), tels que définis dans la norme EN 1822-1. Parmi ceux-ci, on peut avantageusement choisir un filtre de type H14, préférentiellement complété par un ou plusieurs filtres actifs contre les COV (composés organiques volatils) par exemple à base de charbons actifs, de plasma froid, de céramiques poreuses ou autre.

Avec un filtre HEPA de type H14, on dispose avantageusement d'un filtre absolu que l'on peut qualifier préalablement à l'utilisation du système d'exposition aux allergènes 1 par un test d'intégrité selon la norme 14644-3.

Le dispositif de filtration 22 comprend préférentiellement un caisson filtrant encastrable, qui se trouve préférentiellement encastré dans l'entrée d'air 19 de la chambre de mélange 9 et se présente par exemple sous la forme d'une plaque épaisse ou d'une cartouche, rectangulaire, ronde ou d'une autre forme appropriée quelconque.

Selon une autre variante non limitative, le dispositif de filtration 22 peut également comprendre, à la place ou en plus du caisson encastrable, un caisson filtrant en gaine, situé à l'intérieur du conduit d'arrivée d'air 21, bien que cela soit moins favorable pour le nettoyage.

La taille du dispositif de filtration 22, notamment la surface filtrante, dépend évidemment du débit du flux d'air 20 entrant et donc du volume de la salle d'exposition 2 à ventiler. En effet, le débit du flux d'air 20 doit être proportionnel au volume de la salle d'exposition 2, afin de garantir un taux de brassage satisfaisant dans la salle d'exposition, et préférentiellement conforme à la norme 14644-1 permettant ainsi d'obtenir pour la salle d'exposition 2 un classement particulaire hors exposition de classe ISO8.

Pour une salle d'exposition 2 de petite taille comme celle représentée sur la figure 5, un dispositif de filtration 22 de petites dimensions sera suffisant, alors qu'un dispositif de filtration 22 plus étendu, tels que par exemple ceux des figures 6 et 7, sera nécessaire pour une grande salle d'exposition 2, notamment pour celle de la figure 1.

Dans la chambre de mélange 9, le flux d'air 20 qui arrive par l'entrée d'air 19 débouche dans une zone d'entrée dont la section est plus importante que celle du conduit d'arrivée d'air 21. Elle constitue ainsi un élargissement 31 par rapport à ce conduit d'arrivée d'air 21, qui provoque une détente par expansion du flux d'air dépourvu d'allergènes 20 lorsqu'il pénètre dans la chambre. La vitesse du flux d'air 20 est donc ralentie.

Le flux d'air 20 ralenti vient ensuite entraîner le flux de particules d'allergènes 18 qui arrive par la ou les entrée(s) d'allergènes 14.

La pression de sortie du flux de particules d'allergènes 18 est préférentiellement prévue supérieure à la pression de sortie du flux d'air 20 afin d'empêcher l'air de rentrer dans le dispositif d'injection d'allergènes 8, le volume d'air entrant dans la chambre de mélange 9 étant cependant largement supérieur à celui des particules d'allergènes.

Du fait de la disposition relative des entrées d'air 19 et d'allergènes 14, de la forme et de la taille limitée de la chambre de mélange 9, de la mise en pression de la chambre de mélange 9 par le flux d'air 20 entrant et de la réduction de vitesse du flux d'air 20 à son entrée dans la chambre de mélange, il se crée au sein de cette chambre de mélange 9 des micro-turbulences 23 symbolisées sur les figures par les petites flèches noires. Ces micro-turbulences permettent de réaliser un mélange doux et satisfaisant du flux de particules d'allergènes 18 avec le flux d'air 20, sans qu'il soit nécessaire d'ajouter un dispositif de brassage tel qu'un ventilateur ou autre.

Pour améliorer encore ce mélange plusieurs aménagements optionnels de la chambre de mélange peuvent être imaginés par l'homme du métier.

La disposition relative de l'entrée d'air 19 et de l'entrée d'allergènes 14 peut par exemple être choisie pour que le flux d'air 20 qui pénètre dans la chambre de mélange 9 à travers l'entrée d'air 19 vienne balayer l'entrée d'allergènes 14 pour entraîner le flux de particules d'allergènes 18 qui pénètre dans la chambre de mélange 9 à travers l'entrée d'allergènes 14.

Selon une variante préférentielle de l'invention, l'entrée d'air 19 et l'entrée d'allergènes 14 sont pour cela disposées à proximité l'une de l'autre et de façon sensiblement perpendiculaire l'une par rapport à l'autre, c'est-à-dire de façon que les flux d'air 20 et de particules d'allergènes 18, qui débouchent respectivement de l'entrée d'air 19 et de l'entrée d'allergène 14, soient sensiblement perpendiculaires l'un par rapport à l'autre.

En outre, des ailettes 24, nervures, parois, chicanes ou tout autre moyen adapté de canalisation ou de déviation permettant d'orienter les flux à l'intérieur de la chambre de mélange 9 peuvent être prévus dans la chambre de mélange 9, par exemple au niveau de l'entrée d'air 19, pour améliorer la formation des micro-turbulences. Ces dispositifs sont toutefois préférentiellement choisis et disposés de manière à ne pas endommager les particules d'allergènes.

Ainsi par exemple, sur la variante représentée sur la figure 7, les parois 13 latérales de la chambre de mélange 9 sont incurvées de manière à former un rétrécissement progressif suivi d'un agrandissement progressif de la largeur de la chambre de mélange 9, créant ainsi une zone d'étranglement dans laquelle se trouve l'entrée d'allergènes 14.

La totalité du flux d'air 20 entrant dans la chambre à travers le dispositif de filtration 22, et ce même si ce dernier présente une superficie importante, est ainsi dirigé vers l'entrée d'allergènes 14 et passe au-dessus de l'ouverture 15 provoquant l'entraînement du flux d'allergènes 18.

En outre, l'effet successif de la compression due au rétrécissement, puis de la détente causée par l'élargissement subséquent, favorise l'apparition de micro-turbulences 23 dans la deuxième partie de la chambre de mélange 9 qui améliore l'homogénéisation du mélange entre l'air et les particules d'allergènes.

Selon l'invention, la chambre de mélange 9 comporte également au moins une bouche de diffusion 25 qui communique directement ou indirectement avec la salle d'exposition 2 et qui permet d'envoyer vers la salle d'exposition 2 un flux d'air chargé en particules d'allergènes 26 obtenu après mélange dans la chambre de mélange 9 du flux d'air 20 et du flux de particules d'allergènes 18.

Cette bouche de diffusion 25 peut être une simple ouverture ménagée dans la paroi 13 de la chambre de mélange 9 qui débouche directement dans la salle d'exposition 2 ou dans un conduit ouvert allant de la chambre de mélange 9 à la salle d'exposition 2 dans le cas où ces deux zones seraient éloignées l'une de l'autre.

Elle peut également être équipée d'une grille 27 ou de tout autre dispositif approprié permettant d'orienter ou de régler le flux d'air chargé en particules d'allergènes 26 sans le casser, ni endommager les particules d'allergènes.

Le nombre, la taille, la disposition et l'orientation de ces bouches de diffusion 25 dépendent de la taille, de la capacité d'accueil et de la forme de la salle d'exposition 2. La ou les bouches de diffusion 25 sont disposées de manière à assurer une distribution homogène du flux d'air chargés en particules d'allergènes 26 dans toute la salle d'exposition 2, sans direction préférentielle ni zone privilégiée.

Dans certaines variantes, la section de la chambre de mélange 9 peut préférentiellement diminuer de manière à former un rétrécissement 32 juste avant la ou des bouches de diffusion 25. Le flux d'air chargé en particules d'allergènes 26 est ainsi accéléré de nouveau avant de pénétrer dans la salle d'exposition 2 afin d'améliorer le portage des particules d'allergènes dans toute la salle d'exposition.

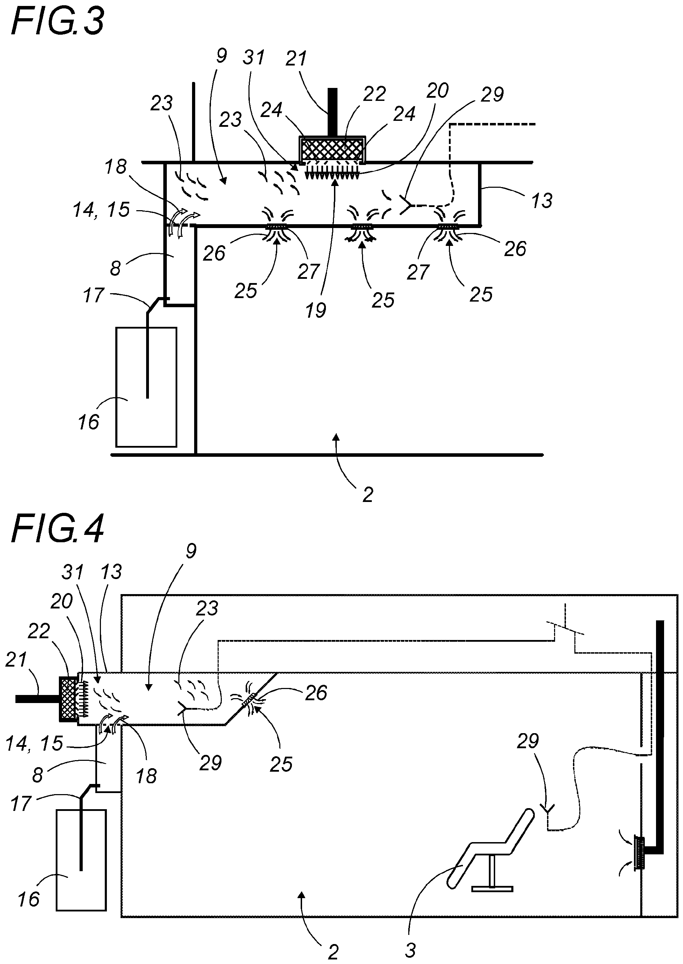

Sur l'exemple de réalisation représenté sur les figures 4 et 5, la chambre de mélange 9 est située dans un faux-plafond, partiellement au-dessus de de la salle d'exposition 2 et par exemple de la salle de contrôle 6.

Elle est ainsi directement en communication avec le dispositif d'injection d'allergènes 8 situé dans la salle de contrôle 6, au moyen d'une ouverture circulaire 15 s'ouvrant dans la paroi de fond de la chambre de mélange 9 et qui sert d'entrée d'allergènes 14.

L'entrée d'air 19 est disposée dans la paroi arrière adjacente de la chambre 9, à proximité de l'entrée d'allergènes 14 et est orientée de façon sensiblement perpendiculaire à celle-ci.

La chambre de mélange 9 est séparée de la salle d'exposition 2 par trois pans de paroi 13, inclinés et orientés dans trois directions différentes de manière à faire face à toutes les zones de la salle d'exposition 2. Elle comporte trois bouches de diffusion 25, qui sont situées une sur chacun de ces pans de paroi 13 et qui sont orientées chacune en direction de l'un des trois groupes de sièges 3.

Le mode de réalisation de la figure 7 comporte quant-à-lui quatre bouches de diffusion 25, plus petites mais orientées selon quatre directions différentes.

Selon l'application visée, l'homme du métier pourra librement définir le nombre et la disposition les plus appropriés pour ces bouches de diffusion 25, en fonction des paramètres géométriques de la salle d'exposition 2 à ventiler, de façon à garantir une diffusion homogène de l'air chargé en allergènes dans la salle d'exposition 2, sans direction préférentielle.

Selon une variante préférentielle de l'invention, la chambre de mélange 9 peut contenir en outre un ou plusieurs capteurs 29 de nature appropriée quelconque, tels que par exemple un capteur de température, de pression ou d'humidité ou un capteur relié à un dispositif de mesure 30 servant par exemple à mesurer la concentration ou la taille des particules d'allergènes, ou tout autre capteur utile pour surveiller, contrôler ou piloter le fonctionnement du système d'exposition aux allergènes 1 selon l'invention.

Avec le système d'exposition aux allergènes 1 de l'invention, le mélange entre les particules d'allergènes et l'air de ventilation dépourvu d'allergènes n'est pas réalisé directement dans la salle d'exposition 2 ni dans le conduit d'arrivée d'air 21, mais dans la chambre de mélange 9 qui est un endroit séparé et distinct.

Seul le flux d'air chargé en particules d'allergènes pénètre dans la salle d'exposition 2. Ce flux est remarquablement homogène et peut être contrôlé à la sortie de la chambre de mélange 9 au moyen d'un ou plusieurs capteurs 29.

Grâce à l'invention, le flux d'air qui est inhalé par les patients se trouvant dans la salle d'exposition, présente avantageusement une très bonne homogénéité avec une concentration en allergènes sensiblement constante avec une tolérance de 20% maximum. Un tel résultat ne peut être obtenu avec aucun des systèmes connus de l'art antérieur.

De manière évidente, l'invention ne se limite pas aux modes de réalisation préférentiels décrits précédemment et représentés sur les différentes figures, l'homme du métier pouvant y apporter de nombreuses modifications et imaginer d'autres variantes sans sortir ni de la portée, ni du cadre de l'invention définis par les revendications.

## Revendications

1. Système d'exposition aux allergènes (1) comprenant un dispositif d'injection d'allergènes (8), un conduit d'arrivée d'air (21) et une salle d'exposition (2) qui contient de l'air chargé en particules d'allergènes obtenu par mélange d'un flux de particules d'allergènes (18) provenant du dispositif d'injection d'allergènes (8) et d'un flux d'air (20) dépourvu d'allergènes amené par le conduit d'arrivée d'air (21), cette salle d'exposition (2) étant destinée à accueillir des patients venant inhaler l'air chargé en particules d'allergènes en vue d'une provocation allergique, système d'exposition aux allergènes **caractérisé :**
**en ce que** le dispositif d'injection d'allergènes (8) est un atomiseur par ondes capillaires qui produit un flux de particules d'allergènes (18) sous la forme d'un nébulisat ;
**en ce que** le système d'exposition comprend une chambre de mélange (9), délimitée par des parois (13), distincte du conduit d'arrivée d'air (21) et de la salle d'exposition (2), dans laquelle se produit ledit mélange entre le flux de particules d'allergènes (18) et le flux d'air (20) dépourvu d'allergènes ;
**en ce que** ladite chambre de mélange (9) comporte :
- au moins une entrée d'allergènes (14), qui communique avec la sortie du dispositif d'injection d'allergènes (8) et par laquelle pénètre le flux de particules d'allergènes (18),
- au moins une entrée d'air (19), qui communique avec le conduit d'arrivée d'air (21) et par laquelle pénètre le flux d'air (20) dépourvu d'allergènes,
- au moins une bouche de diffusion (25) qui communique avec la salle d'exposition (2) et par laquelle s'échappe en direction de la salle d'exposition (2) un flux d'air chargé en particules d'allergènes (26), obtenu par le mélange dans la chambre de mélange (9) du flux d'air (20) dépourvu d'allergènes et du flux de particules d'allergènes (18) ;
et **en ce que** la chambre de mélange (9) comporte une zone d'entrée dont la section est plus importante que celle du conduit d'arrivée d'air (21) et dans laquelle débouche le flux d'air (20) dépourvu d'allergènes qui arrive par l'entrée d'air (19), ce qui provoque une expansion du flux d'air (20) dépourvu d'allergènes lorsqu'il pénètre dans la chambre (9).

2. Système d'exposition aux allergènes (1) selon la revendication 1 **caractérisé en ce que**, juste avant la bouche de diffusion (25), la chambre de mélange (9) comporte une zone (32) dont la section diminue, ce qui accélère le flux d'air chargé en particules d'allergènes (26) avant son entrée dans la salle d'exposition (2).

3. Système d'exposition aux allergènes (1) selon l'une quelconque des revendications précédentes **caractérisé en ce que** l'au moins une entrée d'allergènes (14) et l'au moins une entrée d'air (19) de la chambre de mélange sont disposées l'une par rapport à l'autre de façon que le flux d'air (20) qui pénètre dans la chambre de mélange (9) à travers l'au moins une entrée d'air (19) vienne balayer cette au moins une entrée d'allergènes (14) pour entraîner le flux de particules d'allergènes (18) qui pénètre dans la chambre de mélange (9) à travers l'au moins une entrée d'allergènes (14).

4. Système d'exposition aux allergènes (1) selon l'une quelconque des revendications précédentes **caractérisé en ce que** l'au moins une entrée d'allergènes (14) et l'au moins une entrée d'air (19) de la chambre de mélange sont disposées de façon sensiblement perpendiculaire l'une par rapport à l'autre.

5. Système d'exposition aux allergènes (1) selon l'une quelconque des revendications précédentes **caractérisé en ce que** l'au moins une entrée d'allergènes (14) communique avec la sortie du dispositif d'injection d'allergènes (8) directement ou au moyen d'un conduit droit de faible longueur.

6. Système d'exposition aux allergènes (1) selon l'une quelconque des revendications précédentes **caractérisé en ce que** l'au moins une entrée d'allergènes (14) est un ensemble d'une ou plusieurs ouvertures (15) simples.

7. Système d'exposition aux allergènes (1) selon l'une quelconque des revendications précédentes **caractérisé en ce qu'**il comporte en outre un dispositif de filtration (22), disposé au niveau de l'au moins une entrée d'air (19), ou interposé entre l'au moins une entrée d'air (19) et le conduit d'arrivée d'air (21), ou placé dans le conduit d'arrivée d'air (21).

8. Système d'exposition aux allergènes (1) selon l'une quelconque des revendications précédentes **caractérisé en ce que** l'au moins une bouche de diffusion (25) est une ouverture ménagée dans la paroi (13) de la chambre de mélange (9) qui débouche dans la salle d'exposition (2) ou dans un conduit ouvert allant de la chambre de mélange (9) à la salle d'exposition (2).

9. Système d'exposition aux allergènes (1) selon l'une quelconque des revendications précédentes **caractérisé en ce que** l'au moins une bouche de diffusion (25) est équipée d'une grille (27) ou d'un dispositif permettant d'orienter ou de régler le flux d'air chargé en particules d'allergènes (26).

10. Système d'exposition aux allergènes (1) selon l'une quelconque des revendications précédentes **caractérisé en ce que** la chambre de mélange (9) comporte des ailettes (24), des nervures, des parois, des chicanes ou des moyens de canalisation ou de déviation, qui permettent d'orienter les flux à l'intérieur de la chambre de mélange (9).

11. Système d'exposition aux allergènes (1) selon l'une quelconque des revendications précédentes **caractérisé en ce que** la chambre de mélange (9) comporte un rétrécissement progressif suivi d'un agrandissement progressif de sa largeur, créant ainsi une zone d'étranglement dans laquelle se trouve l'au moins une entrée d'allergènes (14).

12. Système d'exposition aux allergènes (1) selon l'une quelconque des revendications précédentes **caractérisé en ce que** la chambre de mélange (9) contient au moins un capteur (29) de température, de pression, d'humidité ou de mesure de la concentration ou de la taille des particules d'allergènes.

13. Système d'exposition aux allergènes (1) selon l'une quelconque des revendications précédentes **caractérisé en ce que** les parois (13) de la chambre de mélange (9) sont réalisées à partir d'une matière ne relarguant pas ou peu les particules.

14. Système d'exposition aux allergènes (1) selon l'une quelconque des revendications précédentes **caractérisé en ce que** la chambre de mélange (9) est située au moins partiellement au-dessus de la salle d'exposition (2).

## Patentansprüche

1. Allergenexpositionssystem (1) mit einer Allergen- Einspritzvorrichtung (8), einer Lufteinlassleitung (21) sowie einem Expositionsraum (2), der mit Allergenpartikeln belastete Luft enthält, die durch Mischung eines AllergenpartikelStroms (18) aus der Allergen- Einspritzvorrichtung (8) und einem allergenfreien Luftstrom (20), der von der Lufteinlassleitung (21) zugeführt wird, hergestellt wird, dieser Expositionsraum (2) ist für den Aufenthalt von Patienten vorgesehen, die mit Allergenpartikeln belastete Luft inhalieren sollen, um eine allergische Reaktion zu provozieren, Allergenexpositionssystem **dadurch gekennzeichnet,**
**dass** es sich bei der Allergen- Einspritzvorrichtung (8) um einen Kapillarwellen-Zerstäuber handelt, der einen Strom von Allergenpartikeln (18) in Form eines Sprühnebels erzeugt;
dadurch, dass das Expositionssystem eine Mischkammer (9), umfasst, die von Wänden (13) umschlossen wird, getrennt von der Lufteinlassleitung (21) und dem Expositionsraum (2), in der diese Mischung aus dem Allergenpartikelstrom (18) und dem allergenfreien Luftstrom (20) hergestellt wird;
dadurch, dass diese Mischkammer (9) umfasst:
- mindestens einen Allergeneinlass (14), der mit dem Auslass der Allergen-Einspritzvorrichtung (8) in Verbindung steht und durch den der Allergenpartikelstrom (18) fließt,
- mindestens einen Lufteinlass (19), der mit der Lufteinlassleitung (21) in Verbindung steht und durch den der allergenfreie Luftstrom (20) fließt,
- mindestens eine Verteilerdüse (25), die mit dem Expositionsraum (2) in Verbindung steht und aus der in Richtung Expositionsraum (2) ein mit Allergenpartikeln (26) belasteter Luftstrom strömt, erhalten durch Mischung in der Mischkammer (9) des allergenfreien Luftstroms (20) und des Allergenpartikelstroms (18) ;
dadurch, dass die Mischkammer (9) einen Einlassbereich enthält, dessen Querschnitt größer ist als der der Lufteinlassleitung (21) und in dem der allergenfreie Luftstrom (20) mündet, der aus dem Lufteinlass (19) strömt, was zu einer Expansion des allergenfreien Luftstroms (20) führt, sobald er in die Kammer gelangt (9).

2. Allergenexpositionssystem (1) nach Anspruch 1, **dadurch gekennzeichnet, dass** unmittelbar vor der Verteilerdüse (25) die Mischkammer (9) einen Bereich (32) enthält, dessen Querschnitt sich verjüngt, was den mit Allergenpartikeln belasteten Luftstrom (26) beschleunigt, bevor er in den Expositionsraum (2) gelangt.

3. Allergenexpositionssystem (1) nach irgendeinem der vorangehenden Ansprüche, **dadurch gekennzeichnet, dass** der mindestens eine Allergeneinlass (14) und der mindestens eine Lufteinlass (19) der Mischkammer einander gegenüber angeordnet sind, so dass der Luftstrom (20) der in die Mischkammer (9) über den mindestens einen Lufteinlass (19) gelangt, den mindestens einen Allergeneinlass (14) durchbläst, um den Allergenpartikelstrom (18) nach sich zu ziehen, der in die Mischkammer (9) über den mindestens einen Allergeneinlass (14) einströmt.

4. Allergenexpositionssystem (1) nach irgendeinem der vorangehenden Ansprüche, **dadurch gekennzeichnet, dass** der mindestens eine Allergeneinlass (14) und der mindestens eine Lufteinlass (19) der Mischkammer im Wesentlichen senkrecht zueinander angeordnet sind.

5. Allergenexpositionssystem (1) nach irgendeinem der vorangehenden Ansprüche, **dadurch gekennzeichnet, dass** der mindestens eine Allergeneinlass (14) direkt oder über eine kurze gerade Leitung mit dem Auslass der Allergen- Einspritzvorrichtung (8) in Verbindung steht.

6. Allergenexpositionssystem (1) nach irgendeinem der vorangehenden Ansprüche, **dadurch gekennzeichnet, dass** der mindestens eine Allergeneinlass (14) eine Gruppe aus einer oder mehreren einfachen Öffnungen (15) ist.

7. Allergenexpositionssystem (1) nach irgendeinem der vorangehenden Ansprüche, **dadurch gekennzeichnet, dass** es außerdem eine Filtervorrichtung (22) enthält, die in Höhe des mindestens einen Lufteinlasses (19) angeordnet ist oder sich zwischen dem mindestens einen Lufteinlass (19) und der Lufteinlassleitung (21) befindet oder in der Lufteinlassleitung (21) untergebracht ist.

8. Allergenexpositionssystem (1) nach irgendeinem der vorangehenden Ansprüche, **dadurch gekennzeichnet, dass** die mindestens eine Verteilerdüse (25) eine in der Wand (13) der Mischkammer (9) ausgesparte Öffnung ist, die im Expositionsraum (2) mündet oder in einer offenen Leitung, die von der Mischkammer (9) zum Expositionsraum (2) führt.

9. Allergenexpositionssystem (1) nach irgendeinem der vorangehenden Ansprüche, **dadurch gekennzeichnet, dass** die mindestens eine Verteilerdüse (25) mit einem Gitter (27) oder einer Vorrichtung ausgerüstet ist, die eine Ausrichtung oder Steuerung des mit Allergenpartikeln belasteten Luftstroms (26) erlaubt.

10. Allergenexpositionssystem (1) nach irgendeinem der vorangehenden Ansprüche, **dadurch gekennzeichnet, dass** die Mischkammer (9) Lamellen (24), Rippen, Wände, Schikanen oder Mittel zur Kanalisierung oder Ableitung enthält, mit denen der Strom innerhalb der Mischkammer (9) orientiert werden kann.

11. Allergenexpositionssystem (1) nach irgendeinem der vorangehenden Ansprüche, **dadurch gekennzeichnet, dass** die Mischkammer (9) eine progressive Verjüngung aufweist, gefolgt von einer progressiven Verbreiterung und so einen verengten Bereich aufweist, in dem sich der mindestens eine Allergeneinlass (14) befindet.

12. Allergenexpositionssystem (1) nach irgendeinem der vorangehenden Ansprüche, **dadurch gekennzeichnet, dass** die Mischkammer (9) mindestens einen Fühler (29) für Temperatur, Druck, Feuchtigkeit oder zur Messung der Konzentration oder Größe der Allergenpartikel enthält.

13. Allergenexpositionssystem (1) nach irgendeinem der vorangehenden Ansprüche, **dadurch gekennzeichnet, dass** die Wände (13) der Mischkammer (9) aus einem Material hergestellt sind, das Partikel nicht oder kaum freisetzt.

14. Allergenexpositionssystem (1) nach irgendeinem der vorangehenden Ansprüche, **dadurch gekennzeichnet, dass** die Mischkammer (9) zumindest teilweise über dem Expositionsraum (2) angeordnet ist.

## Claims

1. Allergen exposure system (1) comprising an allergen injection device (8), an air inlet duct (21) and an exposure room (2) containing an air containing allergen particles obtained by mixing a flow of allergen particles (18) from the allergen injection device (8) and a flow of air (20) devoid of allergens fed in through the air inlet duct (21), this exposure room (2) being designed to accommodate patients inhaling the air containing allergen particles to cause an allergic provocation, said allergen exposure system being **characterized**
**in that** the allergen injection device (8) is a capillary wave atomizer that produces a flow of allergen particles (18) in the form of a nebulisate;
**in that** the exposure system comprises a mixing chamber (9), contained by walls (13), separate from the air inlet duct (21) and the exposure room (2), in which occurs said mixture between the flow of allergen particles (18) and the flow of air (20) devoid of allergens;
**in that** the said mixing chamber (9) comprises:
- at least one allergen inlet (14), connected to the outlet of the allergen injection device (8) and through which the flow of allergen particles (18) penetrates,
- at least one air inlet (19), connected to the air inlet duct (21) and through which the flow of air (20) devoid of allergens penetrates,
- at least one diffusion outlet (25) connected to the exposure room (2) and through which a flow of air containing allergen particles (26), obtained by mixing in the mixing chamber (9) the flow of air (20) devoid of allergens and the flow of allergen particles (18), escapes towards the exposure room (2);
and **in that** the mixing chamber (9) comprises an inlet zone having a larger section than that of the air inlet duct (21) and into which the flow of air (20) devoid of allergens entering through the air inlet (19) emerges, causing an expansion of the flow of air (20) devoid of allergens when it enters the chamber (9).

2. Allergen exposure system (1) according to claim 1 **characterized in that**, just before the diffusion outlet (25), the mixing chamber (9) comprises a zone (32) with a decreasing section, which accelerates the flow of air containing allergen particles (26) before it penetrates into the exposure room (2).

3. Allergen exposure system (1) according to one of the previous claims, **characterized in that** at least one allergen inlet (14) and at least one air inlet (19) of the mixing chamber are arranged with respect to one another so that the flow of air (20) penetrating into the mixing chamber (9) through at least one air inlet (19) sweeps said at least one allergen inlet opening (14) to carry the flow of allergen particles (18) that penetrates into the mixing chamber (9) through said at least one allergen inlet (14).

4. Allergen exposure system (1) according to any one of the previous claims, **characterized in that** the at least one allergen inlet (14) and the at least one air inlet (19) of the mixing chamber are arranged substantially perpendicularly with respect to each other.

5. Allergen exposure system (1) according to any one of the previous claims **characterized in that** the at least one allergen inlet (14) is connected to the outlet of the allergen injection device (8) directly or by a short straight duct.

6. Allergen exposure system (1) according to any one of the previous claims **characterized in that** the at least one allergen inlet (14) is a set of one or several simple openings (15).

7. Allergen exposure system (1) according to any one of the previous claims, **characterized in that** it also includes a filtration device (22) arranged at the at least one air inlet (19) or is interposed between the at least one air inlet (19) and the air inlet duct (21) or is placed in the air inlet duct (21).

8. Allergen exposure system (1) according to any one of the previous claims, **characterized in that** the at least one diffusion outlet (25) is an opening made in the wall (13) of the mixing chamber (9) opening out into the exposure room (2) or into an open duct extending from mixing chamber (9) to exposure room (2).

9. Allergen exposure system (1) according to any one of the previous claims **characterized in that** the at least one diffusion outlet (25) is provided with a grille (27) or a device for directing or adjusting the flow of air containing the allergen particles (26).

10. Allergen exposure system (1) according to any one of the previous claims, **characterized in that** the mixing chamber (9) comprises fins (24), ribs, walls, baffles or channelling or deviating means, for directing the flows inside the mixing chamber (9).

11. Allergen exposure system (1) according to any one of the previous claims, **characterized in that** the mixing chamber (9) comprises a gradual narrowing followed by a gradual widening of its width, thus forming a throttle zone in which there is at least one allergen inlet (14).

12. Allergen exposure system (1) according to any one of the previous claims, **characterized in that** the mixing chamber (9) contains at least one temperature, pressure, humidity sensor (29) or a device for measuring the concentration or the size of the allergen particles.

13. Allergen exposure system (1) according to any one of the previous claims **characterized in that** the walls (13) of the mixing chamber (9) are made of a material which does not release particles or releases few particles.

14. Allergen exposure system (1) according to any one of the previous claims **characterized in that** the mixing chamber (9) is situated at least partially above the exposure room (2).
